# EUROPEAN PATENT SPECIFICATION

(11) **EP 3 291 240 B1**
(45) Date of publication and mention of the grant of the patent: **10.11.2021**
(21) Application number: 17275134.9
(22) Date of filing: 04.09.2017
(51) Int. Cl.: G16H 20/30, A61H 9/00

(54) **HFCC THERAPY SYSTEM PROVIDING PATIENT INTERVENTIONS FOR IMPROVED ADHERENCE**
HFCC-THERAPIESYSTEM ZUR BEREITSTELLUNG VON PATIENTENEINGRIFFEN ZUR VERBESSERTEN EINHALTUNG
SYSTÈME DE THÉRAPIE HFCC FOURNISSANT DES INTERVENTIONS DE PATIENTS POUR UNE ADHÉRENCE AMÉLIORÉE

(30) Priority: 02.09.2016 US 201615255670
(43) Date of publication of application: 07.03.2018
(73) Proprietor: Respiratory Technologies, Inc., St Paul, MN 55113 (US)
(72) Inventor: ROCK, John, Minneapolis, MN Minnesota 55405 (US); HANSEN, Gary, Inver Grove Heights, MN Minnesota 55076 (US); BUEHLER, Robert, Wayzata, MN Minnesota 55391 (US); CLAPP, Robert, Minneapolis, MN Minnesota 55415 (US); COLSCH, Chad, Minnetonka, MN Minnesota 55345 (US)
(74) Representative: Philips Intellectual Property & Standards

(56) References cited:
- WO-A2-2007/106804
- US-A1- 2007 239 087
- US-A1- 2011 125 068
- US-A1- 2016 151 232

## Description

### FIELD OF THE INVENTION

**2.** The present disclosure relates to high frequency chest compression (HFCC) and high frequency chest wall oscillation (HFCWO) therapy systems and methods. Particularly, the present disclosure relates to HFCC and HFCWO systems and methods for providing patient interventions to improve treatment adherence.

### BACKGROUND OF THE INVENTION

**3.** The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

**4.** A variety of chronic lung conditions, including cystic fibrosis, bronchiectasis, chronic obstructive pulmonary disease (COPD), chronic bronchitis, primary ciliary dyskinesia, a variety of pulmonary conditions, including those resulting from neuromuscular disorders, and many other conditions can cause or have the potential to lead to excess secretions in the lungs. Treatment for clearing or preventing excess secretions can employ various therapies, including nebulizer therapies, mechanical airway clearance therapies, and/or other therapies.

**5.** Mechanical airway clearance therapies conventionally included manual percussion techniques to aid in the clearance of mucus. However, a variety of chest wall oscillation and chest compression devices have been developed to deliver HFCC/HFCWO (referred to herein collectively as HFCC) therapy to a patient. HFCC therapies can aid in the clearance of excess mucus in airways by both mechanically moving the mucus and/or affecting the viscosity of the mucus. Use of HFCC devices is associated with significant reductions in respiratory related hospitalizations, reduced antibiotic use, and dramatic increases in users' assessments of their overall respiratory health and ability to clear their lungs. Such devices typically include the use of an air delivery device in combination with a patient-worn vest. The inflatable vest is linked to an air pulse generator that provides air pulses to the vest during inspiration and/or expiration. The air pulses produce sheer forces within the airways that loosen secretions and also change the viscosity and hydration characteristics of the airway mucous so that it may be cleared by the user's own ciliary action and coughing.

**6.** WO 2007/106804 A2 describes a therapy system operable to deliver a plurality of respiratory therapies, including among others HFCWO therapy, PEP therapy, nebulizer therapy, and cough assist therapy. An assessment system is included, which is operable to assess the efficacy of at least one of the respiratory therapies.

**7.** US 2016/151232 A1 describes a HFCC therapy system with a garment, an air pulse system operated by a controller, and a display for conveying therapy session or summary data to a user.

**8.** US 2011/125068 A1 describes a HFCC therapy system for deriving one or more frequencies for a therapeutically beneficial treatment protocol based on measurements made via a mouthpiece.

**9.** In many cases, HFCC treatments may be self-administered by a patient using an HFCC device. In other cases, HFCC treatments may be administered with the help of a family member, friend, or caretaker. Some patients may have difficulty adhering to a prescribed or suggested treatment regimen or schedule. For example, some patients may forget to undergo therapy sessions, or may forget or misremember prescribed or suggested therapy parameters. Some patients may lack motivation or become discouraged or frustrated when they do not see improvement after therapy sessions or when they feel discomfort during therapy sessions. Thus there is a need in the art for devices, systems, and methods for providing patient interventions to improve treatment adherence with respect to HFCC therapy.

### BRIEF SUMMARY OF THE INVENTION

**10.** The invention is solely defined in appended independent claims 1 and 6. Particular embodiments are defined in dependent claims.

**11.** According to a first aspect of the present invention, a system is provided for providing high frequency chest compression therapy. The system comprises a patient device comprising a vest configured to be worn by a patient, the vest housing an air bladder. The system also comprises a therapy device flowably coupled to the patient device and providing percussive pulsed air to the patient device. The percussive pulsed air has a pressure and a frequency. The system also comprises a database storing therapy data recorded for one or more therapy sessions. The system further comprises a user interface for accessing therapy data in the database. The system also comprises a controller. The controller comprises a device control module for controlling parameters of the therapy device, the parameters comprising the pressure and frequency of the percussive pulsed air. The controller also comprises a therapy monitoring module for monitoring and recording therapy data during a therapy session, the therapy data comprising air pressure data. The controller also comprises a data analysis module for analyzing the therapy data and configured to determine whether different peak pressures exist when the patient is wearing the patient device and when the patient is not wearing the patient device based on the air pressure data by comparing the recorded therapy data to at least one of expected therapy data, regimented therapy data, historical therapy data, and therapy data for one or more other patients. The controller further comprises an alerts module configured for alerting the patient via the user interface that the therapy is not being received, and/or configured for alerting a healthcare professional that the patient may not be receiving an appropriate therapy, based on a determination result of the data analysis module.

**12.** According to a second aspect of the present invention, a method is provided for increasing patient adherence to a high frequency chest compression therapy regimen. The method comprises the step of recording therapy data for one or more therapy sessions of a patient, the therapy data comprising air pressure data. The method further comprises the step of analyzing the therapy data and determining whether different peak pressures exist when the patient is wearing the patient device and when the patient is not wearing the patient device based on the air pressure data by comparing the recorded therapy data to at least one of expected therapy data, regimented therapy data, historical therapy data, and therapy data for one or more other patients. The method also comprises the step of alerting the patient via the user interface that the therapy is not being received, and/or alerting a healthcare professional that the patient may not be receiving an appropriate therapy, based on a determination result.

**13.** The following presents a simplified summary of one or more embodiments of the present disclosure in order to provide a basic understanding of such embodiments. This summary is not an extensive overview of all contemplated embodiments, and is intended to neither identify key or critical elements of all embodiments, nor delineate the scope of any or all embodiments.

**14.** In some embodiments, the alerts module may be configured for initiating user alerts for providing a social support intervention configured to provide a sharing option whereby the patient can share recorded therapy data. In some embodiments, the system may additionally include a patient device pressure sensor, a spirometer, and/or a pneumotachometer. In some embodiments, the user interface may have a touch screen display. Furthermore, in some embodiments, the recorded therapy data may be communicated to a healthcare professional.

**15.** In some embodiments, the method may include communicating the recorded therapy data to a healthcare professional.

### BRIEF DESCRIPTION OF THE DRAWINGS

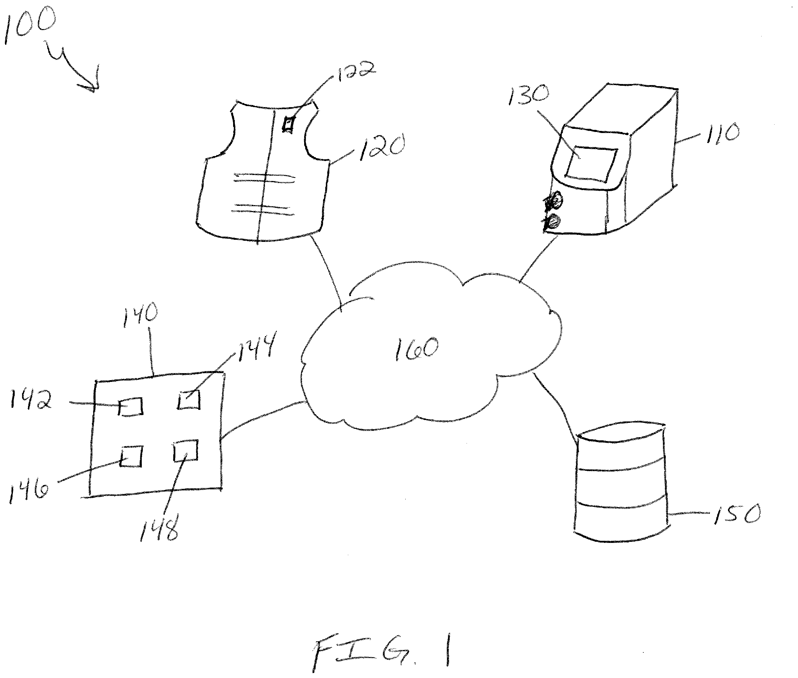

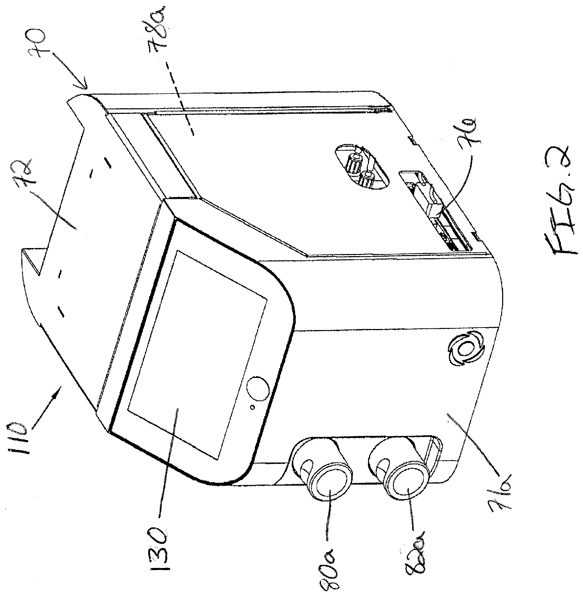

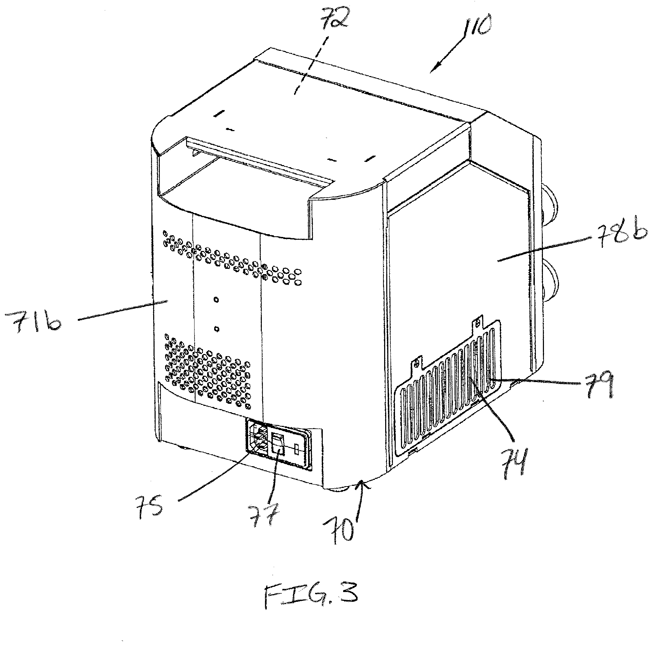

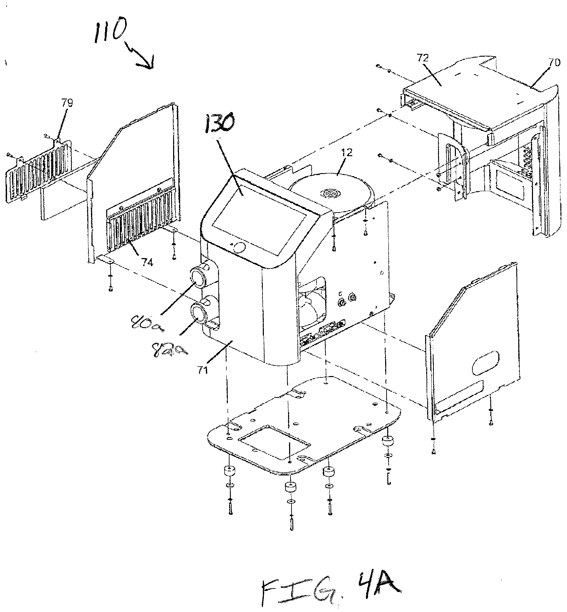

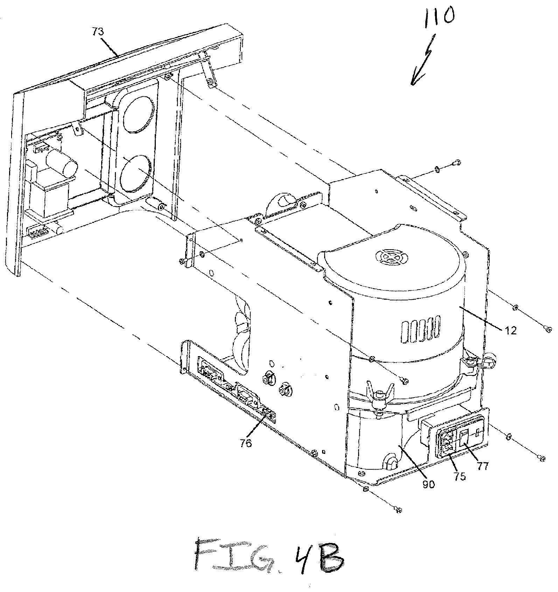

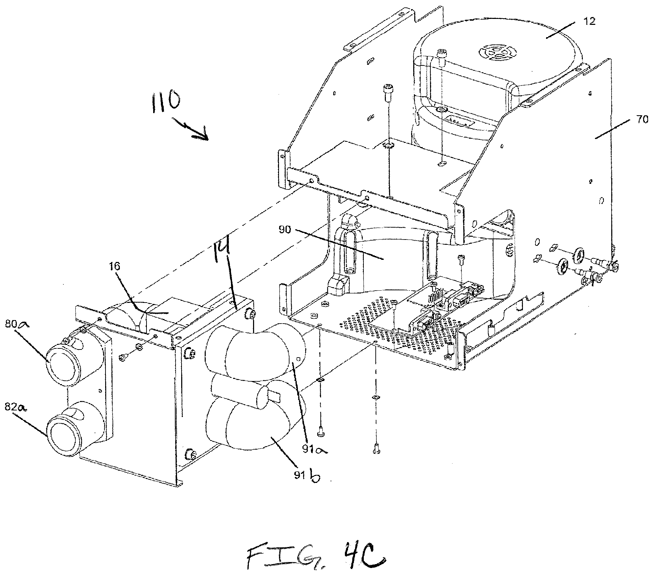

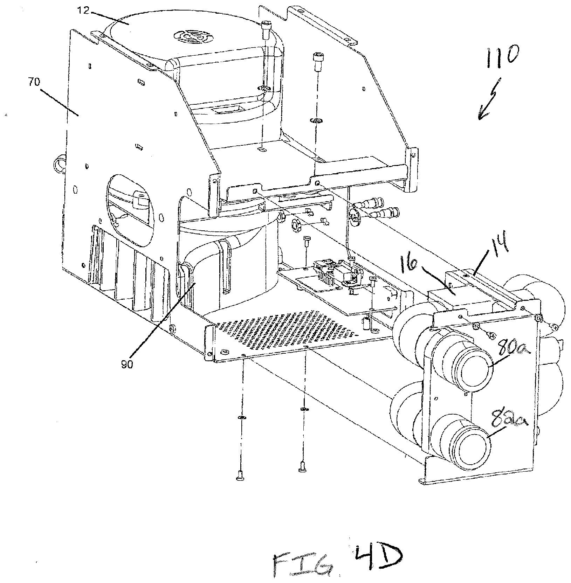

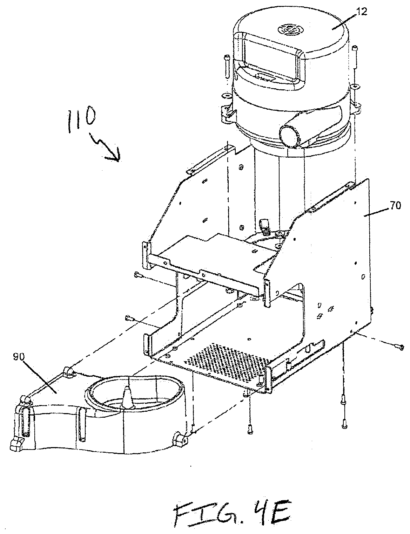

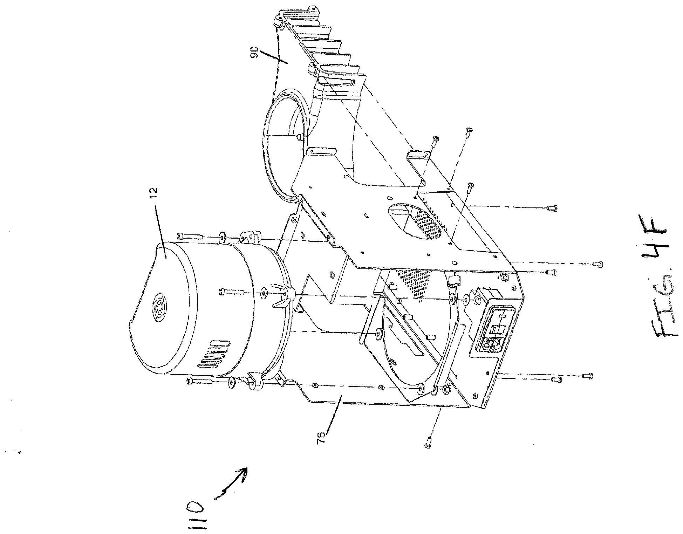

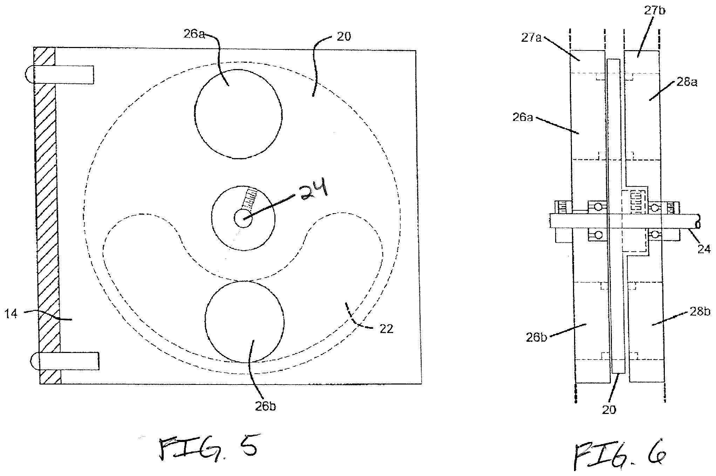

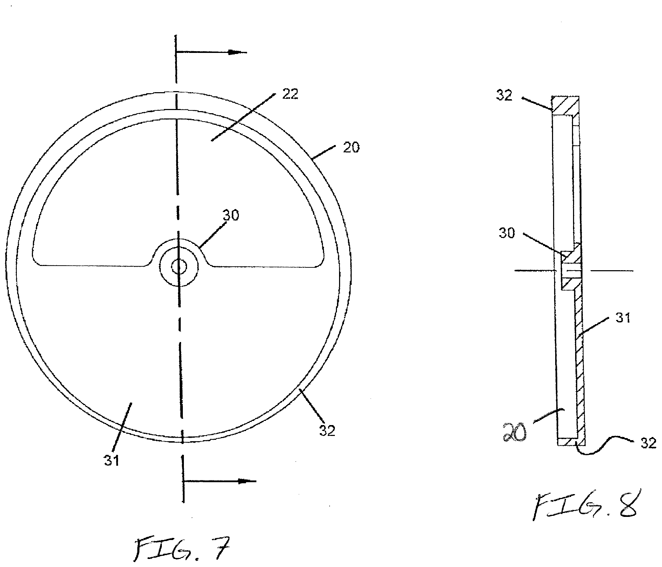

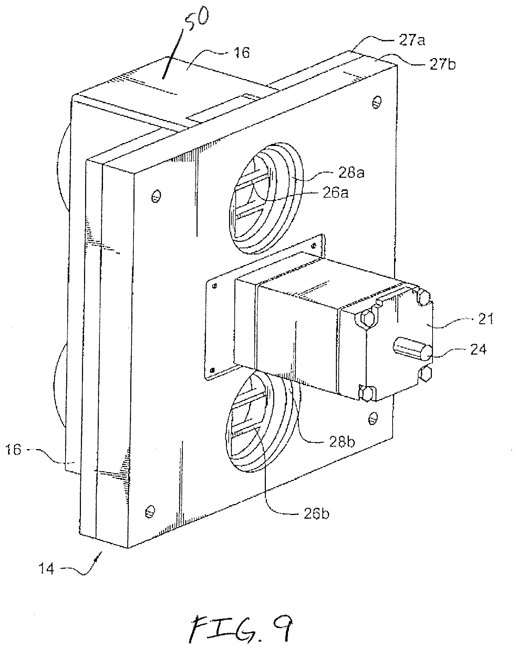

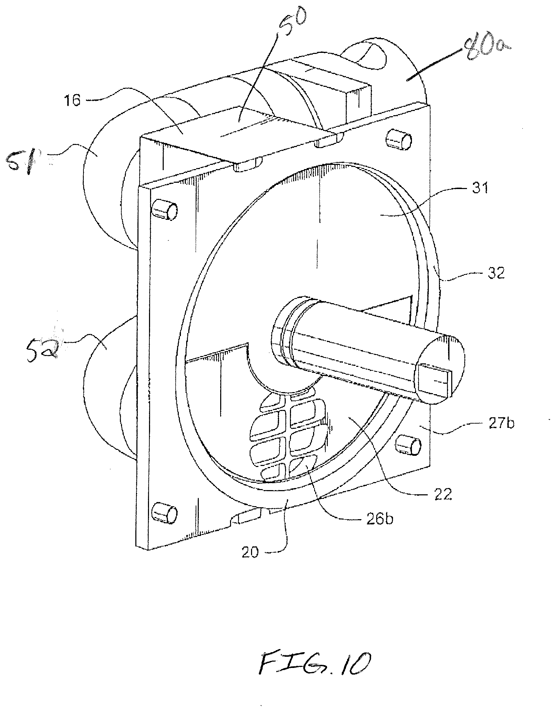

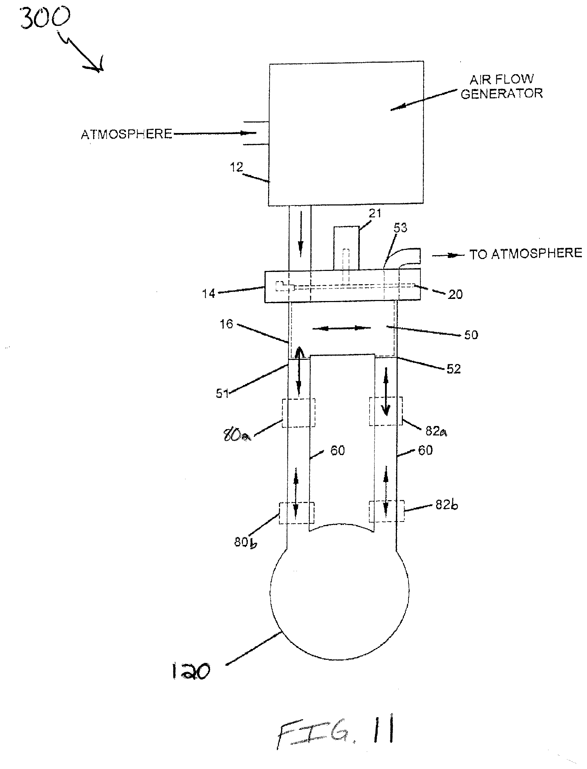

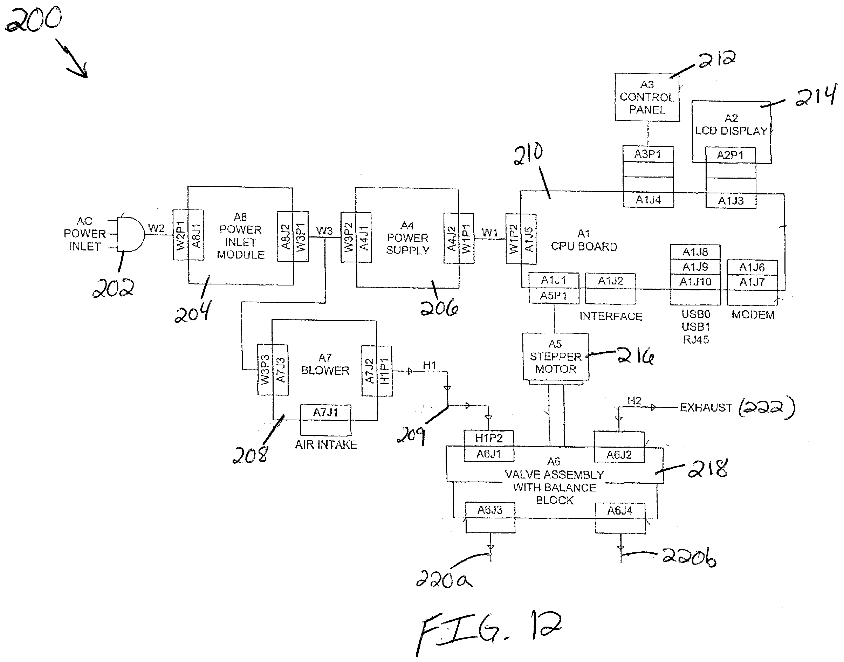

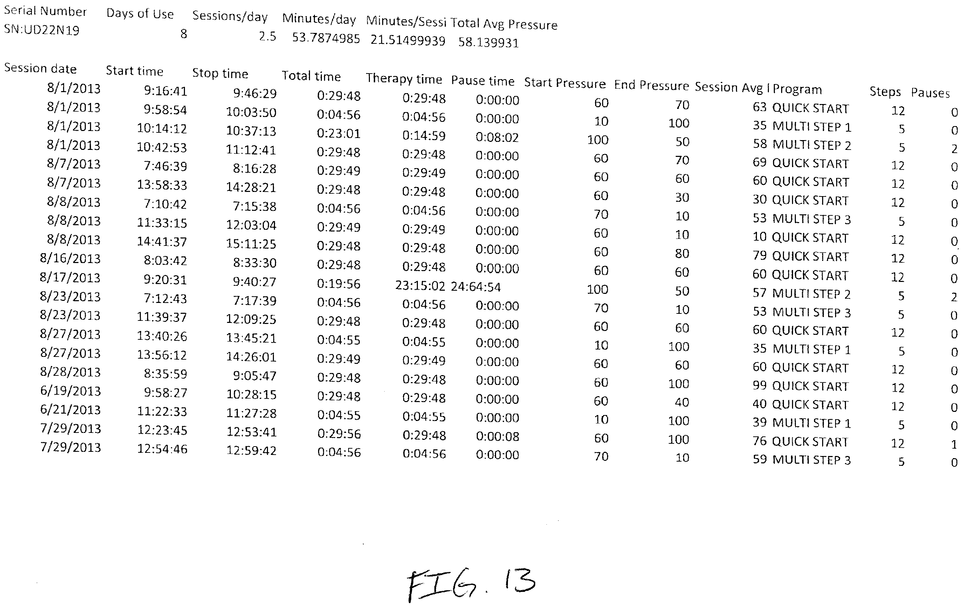

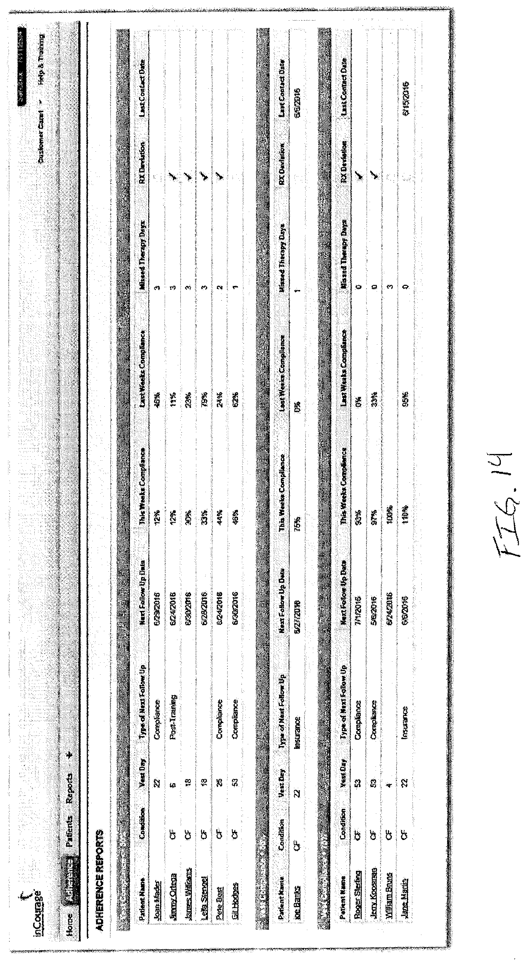

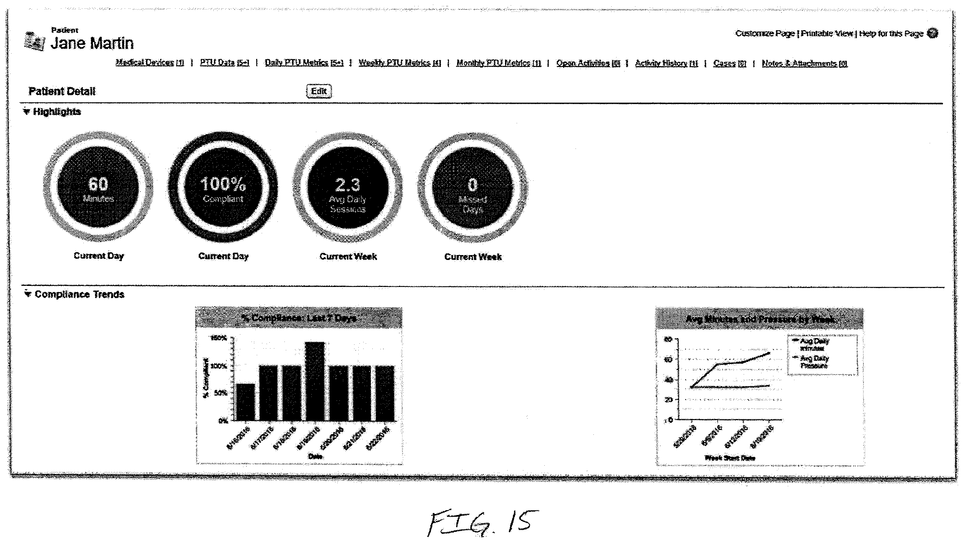

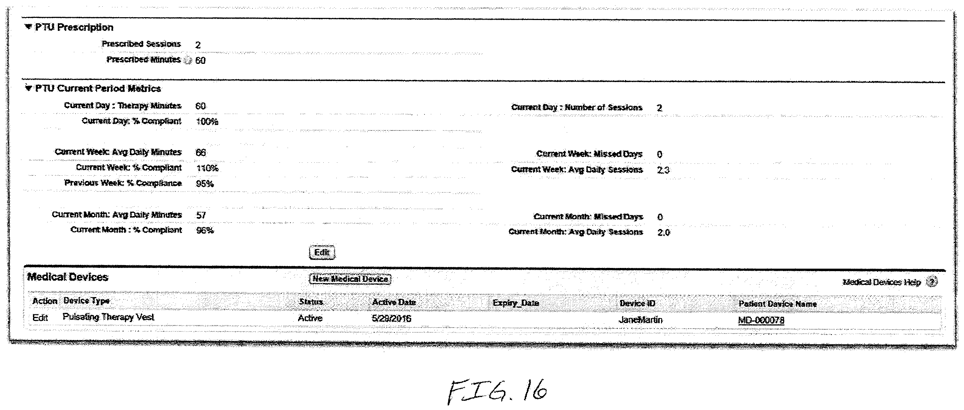

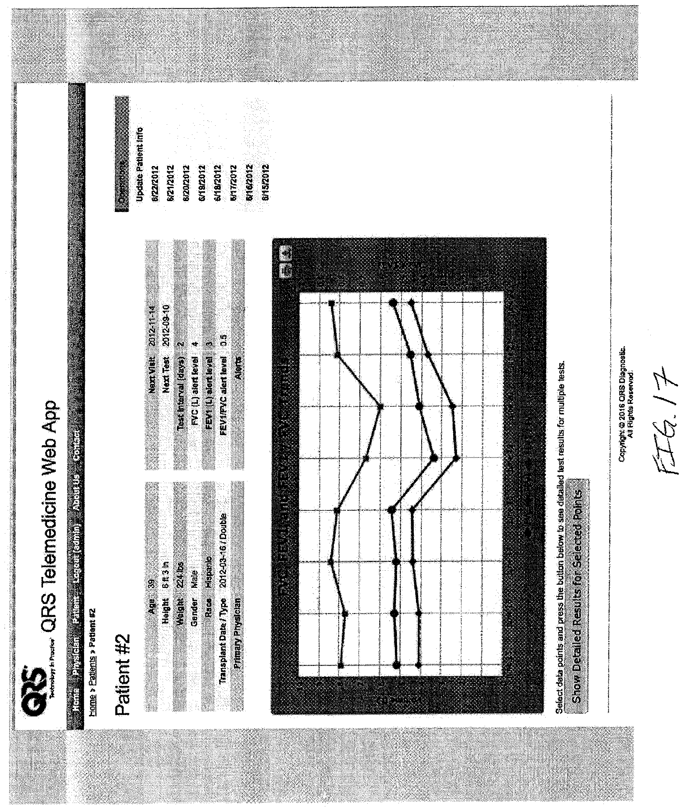

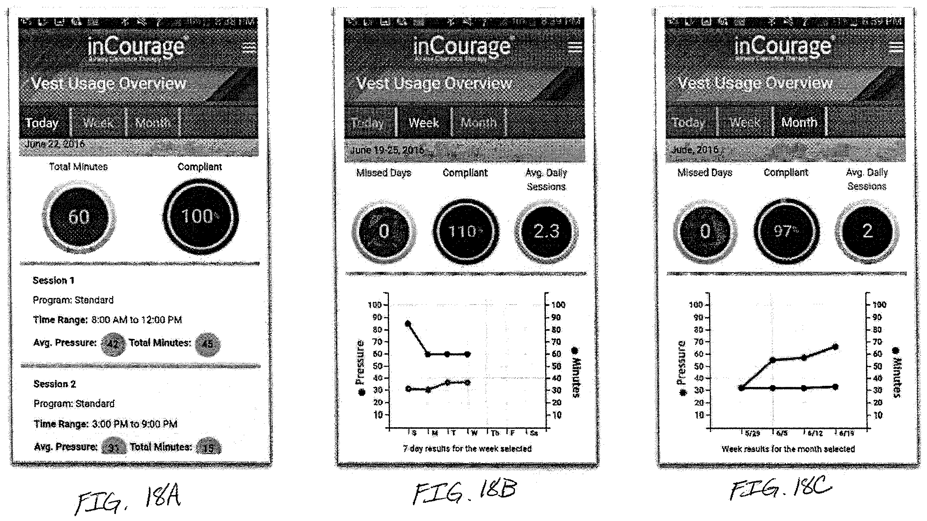

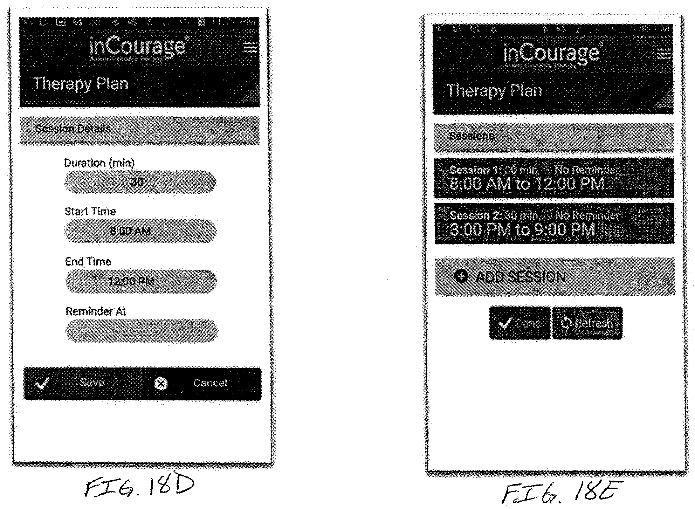

**16.** While the specification concludes with claims particularly pointing out and distinctly claiming the subject matter that is regarded as forming the various embodiments of the present disclosure, it is believed that the invention will be better understood from the following description taken in conjunction with the accompanying Figures, in which:
**17.** FIG. 1 is a schematic diagram of a therapy system of the present disclosure, according to one or more embodiments.
**18.** FIG. 2 is a perspective view of a percussive pulsed air device of the present disclosure, according to one or more embodiments.
**19.** FIG. 3 is another perspective view of the percussive pulsed air device of FIG. 2, according to one or more embodiments.
**20.** FIG. 4A is an exploded view of the percussive pulsed air device of FIG. 2, according to one or more embodiments.
**21.** FIG. 4B is an alternate exploded view of the percussive pulsed air device of FIG. 2, according to one or more embodiments.
**22.** FIG. 4C is an alternate exploded view of the percussive pulsed air device of FIG. 2, according to one or more embodiments.
**23.** FIG. 4D is an alternate exploded view of the percussive pulsed air device of FIG. 2, according to one or more embodiments.
**24.** FIG. 4E is an alternate exploded view of the percussive pulsed air device of FIG. 2, according to one or more embodiments.
**25.** FIG. 4F is an alternate exploded view of the percussive pulsed air device of FIG. 2, according to one or more embodiments.
**26.** FIG. 5 is a front view of a frequency control module of the present disclosure, according to one or more embodiments.
**27.** FIG. 6 is a side view of the frequency control module of FIG. 5, according to one or more embodiments.
**28.** FIG. 7 is a front view of a blade and hub of the present disclosure, according to one or more embodiments.
**29.** FIG. 8 is a side view of the blade and hub of FIG. 7, according to one or more embodiments.
**30.** FIG. 9 is a perspective view of a frequency control module and pressure control device of the present disclosure, according to one or more embodiments.
**31.** FIG. 10 is a perspective view of a portion of the frequency control module and the pressure control device of FIG. 9, according to one or more embodiments.
**32.** FIG. 11 is a flow diagram of a percussive pulsed air device of the present disclosure, according to one or more embodiments.
**33.** FIG. 12 is a schematic diagram of a system of the present disclosure, according to one or more embodiments.
**34.** FIG. 13 is an example of device adherence data recorded by a system of the present disclosure, according to one or more embodiments.
**35.** FIG. 14 is a screenshot from an example of a user interface whereby a user may access adherence data recorded for a plurality of patients, according to one or more embodiments.
**36.** FIG. 15 is a screenshot from another example of a user interface, according to one or more embodiments.
**37.** FIG. 16 is a screenshot from another example of a user interface, according to one or more embodiments.
**38.** FIG. 17 is a screenshot from another example of a user interface, according to one or more embodiments.
**39.** FIG. 18A is a screenshot from an example of a mobile user interface, according to one or more embodiments.
**40.** FIG. 18B is another screenshot from an example of a mobile user interface, according to one or more embodiments.
**41.** FIG. 18C is another screenshot from an example of a mobile user interface, according to one or more embodiments.
**42.** FIG. 18D is another screenshot from an example of a mobile user interface, according to one or more embodiments.
**43.** FIG. 18E is another screenshot from an example of a mobile user interface, according to one or more embodiments.

### DETAILED DESCRIPTION

**44.** The present disclosure relates to devices, systems and methods for improving patient adherence with respect to HFCC therapy. A therapy system of the present disclosure may include a percussive pulsing therapy device configured for delivering pulsating or intermittent airflow to a patient device, such as a patient vest worn by a patient. The airflow delivered to the patient vest may have an air pressure and a frequency, each of which may be adjustable in some embodiments. For example, a user, such as a patient or caregiver, may adjust the air pressure and/or frequency prior to and/or during a therapy session. To receive a treatment, a user may wear the patient vest around his or her chest and turn on the therapy device such that percussive, pulsed air may be delivered to the patient's chest region for the duration of the therapy session. A therapy system of the present disclosure may additionally include a controller, a database, and one or more sensors. The controller and/or one or more sensors may record therapy data for a therapy session. Based on the recorded therapy data, the system may provide encouragement, advice, motivation, and/or options or information to a user by way of skills training, goal setting, feedback, reinforcement, environmental prompts, social support, and/or other patient interventions.

**45.** Turning now to FIG. 1, a percussive pulsing therapy system 100 of the present disclosure is shown. As shown, the system 100 may include a therapy device 110 and a patient device 120. The therapy device 110, which is more thoroughly described below with respect to FIGS. 2-11, may generally be configured to provide percussive air flow to the patient device 120. The system 100 may additionally include one or more sensors 122, a user interface 130, a controller 140, and a database 150, each of which may be communicably coupled to the therapy device 110 over a wired or wireless network 160.

**46.** The patient device 120 may generally be configured to receive percussive air flow received from the therapy device 110, and deliver the airflow to a patient. For example, the patient device 120 may be or include a vest or jacket worn by the patient such that the percussive air may be applied to the patient's chest region. The patient device 110 may be or include a vest similar to those described in U.S. Patent Application No. 13/850,286, entitled "Air Vest," and filed March 25, 2013, hereby incorporated by reference herein in its entirety, or any other vest or patient device described in other patents or applications incorporated herein. The patient for which a device of the present disclosure is used may be a human or other animal. For example, both human and equine applications may be practicable, with differently configured vests being defined by the particular applications.

**47.** The one or more sensors 122 may be configured to sense or measure various parameters. One or more sensors 122 may be arranged on, in, or connected to the patient device 120, as shown for example in FIG. 1. Additionally or alternatively, one or more sensors 122 may be arranged on, in, or connected to the therapy device 110. In still other embodiments, additionally or alternatively, one or more sensors 122 may be a handheld device or a remote device in communication with the system 100 over the network 160. One example of a sensor 122 may be a pressure sensor configured to measure air pressure. The pressure sensor may be configured to measure air pressure within the patient device 120, within the therapy device 110, within an airflow line between the patient device and therapy device, or at any other suitable location within the system 100. Another example of a sensor 122 may be a breath sensor, such as a pneumotachometer, anemometer, microphone, or other suitable breath sensor for measuring inhaled and/or exhaled airflow. Such a breath sensor may be coupled to the patient device 120 or may be a separate device in some embodiments. Other sensors 122 that may be in communication with the therapy device 110 and/or patient device 120 include, but are not limited to, an acoustic sensor for cough and/or noise management; a vibrational sensor, such as a piezoelectric sensor for cough and percussion feedback; an accelerometer for cough, positioning, and/or percussive action; and a thermal sensor for patient comfort. In other embodiments, the system 100 may include other sensors as well. In some embodiments, the system 100 may include leveraging the use of one or more sensors incorporated in another device, such as a smartphone, smart watch, or fitness tracker. For example, the microphone on a smartphone or smart watch may be used to evaluate a user's cough sounds and/or breathing sounds.

**48.** In some embodiments, the user interface 130 may allow a user to interact with the system 100. For example, the user interface 130 may allow a user to view, enter, modify, or delete data. In some embodiments, the user interface may be an integral component of the therapy device 110. For example, the user interface 130 may be arranged on or in, or may be otherwise communicably coupled to, the therapy device 110. The user interface 130 may include a touch screen or other display and/or one or more selectable buttons, keys, or switches. In other embodiments, the user interface 130 may be accessible via a user device, such as a laptop computer, desktop computer, smartphone, smart watch, tablet computer, personal digital assistant, or other user device. In some embodiments, the user interface 130 may be or include a computer program or application, including for example a mobile application. In some embodiments, the user interface 130 may provide for voice activated commands or other user inputs. The user interface 130 may have a plurality of navigable screens, pages, tabs, or other elements in some embodiments. The user interface 130 may be accessible by various users, such as a patient who may wear the patient device 120 or for whom the therapy may be directed, a healthcare professional, a caretaker, a system administrator, or other users. In some embodiments, the user interface 130 may prompt a user for a username, user identifier, and/or password or other identifying or login information before a user can access the controller 140 and/or database 150. In some embodiments, a user may be prompted for login or identifying information prior to beginning a therapy session. This may allow the system 100 to differentiate data for multiple patients, for example, and/or may maintain secure access to patient data.

**49.** The controller 140 may have hardware and/or software for monitoring, processing, analyzing, sending, and/or receiving data. The controller 140 may have one or more modules, such as a device control module 142, a therapy monitoring module 144, a data analysis module 146, and an alerts module 148. In other embodiments, the controller 140 may have alternative, combined or additional modules or other components.

**50.** The device control module 142 may generally control operation of the therapy device 110. The device control module 142 may control the therapy device 110 based on predetermined settings or commands, user selected settings or commands, and/or other inputs. Based on these various inputs, the device control module 142 may operate the timing, pressure settings, frequency settings, pause or rest settings, and/or other operations of the therapy device 110.

**51.** The therapy monitoring module 144 may generally track, monitor, measure, and/or record various parameters related to one or more therapy sessions administered with the therapy device 110. For example, in some embodiments, the therapy monitoring module 144 may record air pressure, frequency, timing, and/or other variables selected, altered, or otherwise used during a therapy session. The therapy monitoring module 144 may additionally record sensed airflow, breath, or other data measured by the one or more sensors 122.

**52.** The data analysis module 146 may generally analyze therapy data tracked, monitored, measured, and/or recorded by the therapy monitoring module 146 and/or data stored in the database 150. For example, therapy data related to more than one therapy session performed over a period of time may be compared or analyzed by averaging or performing other calculations with respect to the data, charts, graphs, tables, and/or other analysis methods. Analysis of the therapy data may be performed automatically or manually. In some embodiments, the analysis module 146 may analyze the therapy data to determine whether the patient is using the patient device 120 and/or therapy device 110 as prescribed or in an effective manner. For example, the data analysis module 146 may compare therapy data from one or more therapy sessions to a prescribed or suggested therapy schedule, prescribed or suggested air pressures, therapy durations, or pulse frequencies, previously measured or expected airflow rates, lookup tables, statistics, and/or other data. The data analysis module 146 may be configured to determine, for example, whether a patient is adhering to a prescribed or suggested treatment routine or schedule, whether the patient is on track to meet one or more therapy goals, whether the patient is using the patient device 110 and/or therapy device 120 effectively, and/or whether the patient may benefit from alternate treatment settings, improved techniques or performance, or other parameters.

**53.** In some embodiments, the data analysis module 146 may perform one or more diagnostic functions. For example, the data analysis module 146 may evaluate the sound of a patient's cough via a cough sensor 122 to determine diagnostic and/or prognostic information related to the patient's particular condition or health. Similarly, diagnostic data may be determined based on a patient's breathing sounds via a breath sensor 122.

**54.** The alerts module 148 may generally send an alert to a user of the therapy device 120, such as a patient, or another individual such as a family member, friend, caretaker, or healthcare professional. In some embodiments, an alert may be sent where, for example, the data analysis module 146 determines that the patient is not adhering to a prescribed or suggested treatment regimen. For example, where a patient misses one or more scheduled therapy sessions, where a therapy session is shorter in duration than prescribed or suggested, and/or where the patient is not using a prescribed or suggested pressure or pulse frequency, the patient, a healthcare professional, and/or other individual may receive an alert indicating the adherence information. In some embodiments, the alerts module 148 may be configured to send an alert to a patient or user as a reminder that it may be time for a scheduled or prescribed treatment. In some embodiments, the alerts module 148 may send alerts during a therapy session. For example, where the data analysis module 146 determines that the patient could benefit from a different pressure setting and/or pulse frequency or an adjustment to the patient device 120, the alerts module 148 may send an alert to the patient. The alerts module 148 may additionally send informational, motivational, or other alerts. For example, the alerts module 148 may alert a patient that he or she has achieved a therapy goal or is close to achieving such a goal. Generally, alerts may be sent over a wired or wireless network to one or more devices. For example, in some embodiments, alerts may be received at the user interface 130. Additionally or alternatively, alerts may be received via a user device such as a desktop computer, laptop computer, smartphone, tablet computer, smart watch, or other device. Alerts may be sent in generally any suitable format, such as an on-screen popup window, an email, a text message, a voice message, a sound, a vibration, and/or other alert formats.

**55.** With continued reference to FIG. 1, the database 150 may be a local or remote database. For example, in some embodiments, the database 150 may be a local database integral to or communicably coupled to the therapy device 110. In other embodiments, the database 150 may be a remote database such as a cloud storage database. Other databases are contemplated as well. In some embodiments, the system 100 may have more than one database 150. The database 150 may store data related to the system 100, including identifiers for the patient device 120 and therapy device 110. The database 150 may store preset or preconfigured device settings in some embodiments, such as a preset progression of air pressures and/or pulse frequencies. The database 150 may additionally store user data, such as user identifiers, personal information, contact information, prescribed therapy data, health history or other health data, and/or other user data. The database 150 may store data related to a patient's prescribed or suggested therapy, such as schedules, and prescribed or suggested air pressures, pulse frequencies, or progressions. Additionally, in some embodiments, the database 150 may store therapy data related to one or more therapy sessions, including for example variables controlled by the device control module 142, such as air pressure and pulse frequency, data sensed by the one or more sensors 122, user inputs or selections, data calculated or derived from sensed data, and/or other therapy data. In some embodiments, the database 150 may store user data and/or adherence data for more than one patient. In some embodiments, the database 150 may store expected, average, or typical therapy data. For example, the database 150 may store one or more lookup tables having expected inhalation, exhalation, or other airflow rates for patients having particular conditions, ages, or other parameters. Data stored in the database 150 may be accessible via the user interface 130. Additionally or alternatively, data in the database 150 may be accessible locally or remotely by download or upload.

**56.** The therapy device 110, shown in detail in FIGS. 2 and 3, may be configured to couple to the patient device 120, which may include a patient vest or jacket, via one or more lines or hoses. The device 110 may have a shell or housing 70 having a front portion 71a, a back portion 71b, a top portion 72, a first side portion 78a, and a second side portion 78b. The housing 70 may be composed of plastic, metal, or other suitable materials, and may generally be configured to house the electrical and mechanical components of the device 110. The device 110 may have one or more ports 80a, 82a for coupling to a patient vest, an air intake 74, one or more electrical connections 75, one or more telecom connections 76, and a power switch 77. As described above, the device 110 may additionally have a user interface 130 in some embodiments.

**57.** The one or more ports 80a, 82a may be configured to couple to the patient device 120. The ports 80a, 82a may generally be configured to allow air to travel between the therapy device 110 and the patient device 120. The ports 80a, 82a may be arranged at any suitable location on the device 110. For example, as shown in FIG. 2, the ports 80a, 82a may be extend through a front portion 71a of the housing 70. In other embodiments, the ports 80a, 82a may be arranged differently on the device 110. The ports 80a, 82a may be or include quick connect air couplings in some embodiments. Each quick connect air coupling may include male and female portions and a latch or other release for quickly disconnecting the portions. The benefits of the quick connect air couplings include minimization of inadvertent air hose disconnects and improved freedom of movement as the locking air coupling permits rotation between the air hose and the patient device 120 or air generator.

**58.** The air intake 74 may be configured to pull atmospheric air into the device 110. The air intake 74 may generally be arranged in any suitable location on the device 110. For example, as shown in FIG. 3, the air intake 74 may be positioned on or through a second side portion 78b of the housing 70. In some embodiments, the device 110 may have a filter 79 arranged over the air intake 74. The filter 79 may be configured to minimize debris entry into the system 110. In some embodiments, the filter 79 may be removable, such that it may be periodically removed, and cleaned and/or replaced.

**59.** The device 110 may have one or more electrical connections 75 and/or one or more telecom connections 76. The connections 75, 76 may provide for coupling one or more devices or components to the device 110 via a wired or wireless connection. For example, a computer, tablet, smartphone, USB device, or other device may connect to the therapy device 110 using a wired or wireless connection. The electrical connection(s) 75 and/or telecom connection(s) 76 may be arranged at any suitable location on the device 110.

**60.** The device 110 may have a power switch 77 for operating the device. The power switch 77 may be arranged at any suitable location on the device 110. As shown, the device 110 may include a power inlet module

**61.** FIGS. 4A-4F illustrate more detailed, internal views of the therapy device 110. As shown, the device 110 may have a plenum 90, an airflow generator 12, a pulse frequency control module 14, and a pressure control unit 16, each arranged within the housing 70.

**62.** The plenum 90 may generally be arranged between the airflow generator 12, such as an intake of the airflow generator, and the air intake 74. The plenum 90 may generally provide an air conduit between the air intake 74 and the airflow generator 12. In some embodiments, the plenum 90 may be configured to reduce sound generation by efficiently or effectively drawing air into the generator 12, as compared with an open fan inlet.

**63.** The airflow generator 12 may be configured to provide airflow to the patient device 120 during a therapy session. The airflow generator 12 may be generally arranged between the air intake 74 (or plenum 90) and the pulse frequency control module 14. In some embodiments, the air flow generator 12 may be or include a compressor in an enclosed compartment having inlet and outlet ports. In other embodiments, the airflow generator 12 may be or include a fan or blower having fixed or variable speed. The airflow generator 12 may generally be controlled to adjust the amplitude of air pulses transmitted from the therapy device 110 to the patient device 120. The airflow generator 12 may be flowably coupled to the pulse frequency control module 14 through ports 91a and 91b. Port 91a may allow airflow from the airflow generator 12 to enter the pulse frequency control module 14, whereas port 91b may allow air to flow from the pulse frequency control module back to the airflow generator and/or out to the atmosphere via an exhaust. It may be appreciated that in other embodiments, ports 91a and 91b may be reversed.

**64.** The pulse frequency control module 14 may be configured to adjust the frequency or speed of the intermittent pulses being delivered to the patient device 120 during a therapy session. FIG. 5 illustrates one embodiment of a pulse frequency control module 14. In some embodiments, the pulse frequency control module 14 may have one or more rotating fan-like blades 20. For example, the module 14 may have a blade 20 with one or more cutout portions 22, and rotatable on a central axis. The blade 20 may be arranged proximate to air ports 26a and 26b. The cutout portions 22 may be configured to move across the air ports 26a, 26b as the blade 20 rotates on its central axis. The blade 20 may be adapted to periodically interrupt an air stream received from the airflow generator 12. During these interruptions, air pressure may build up behind the blade 20. When released, as by the passage of blade 20, the air may travel as a pressure pulse to the patient device 120. The resulting pulses can be in the form of fast rise, triangle wave pressure pulses. Alternative waveforms, such as sinusoidal waveforms, square waveforms, or other waveforms can be defined through alternate forms of cutout portions 22, through accurate control of blade 20, such as via an electronically controlled stepper motor, or by other means. These pulses, in turn, can produce significantly faster air movement in the patient's lungs, in the therapeutic frequency range of about 5 Hz to about 25 Hz, for example, as measured at the patient's mouth. In combination with higher flow rates into the lungs, as achieved using the present apparatus, these factors may result in stronger mucus shear action, and thus more effective therapy in a shorter period of time.

**65.** As shown in FIG. 6, the blade 20 may be retained a centrally located motor driven shaft 24, which may serve to rotate the blade, and in turn, provide airflow access to and through the air ports 26a and 26b. In some embodiments, the motor driven shaft 24 may be controlled by a stepper motor. The blade 20 may be controlled to provide particular pressure waveforms to the patient device 120. As additionally shown in FIG. 6, a pair of plates 27a and 27b may be mounted on an axis concentric with that of motor drive shaft 24, and effectively sandwich the blade assembly between them. The end plates 27a, 27b may have corresponding air ports 26a and 26b (in plate 27a) and 28a and 28b (in plate 27b). The air ports may overlap such that air delivered from the external surface of either end plate 27a, 27b via an air port may be free to exit the corresponding air port in the opposite plate, at such times as the blade cutout portion 22 of the valve blade 20 is itself in an overlapping position therebetween. By virtue of the rotation of cutout portions 22 past the overlapping air ports 26a, 26b, in the course of constant air delivery from one air port toward the other, the rotating fan blade 20 may effectively function as a valve to permit air to pass into the corresponding air port in a semi-continuous and controllable fashion. The resultant delivery may take a sinusoidal, or in some cases triangular, wave form, by virtue of the shape and arrangement of the fan blade cutout portions 22.

**66.** The pulse frequency control module 14 may be adapted (e.g., by configuring the dimensions, pitch, etc. of one or more fan blades) to provide wave pulses in a variety of forms, including sine waves, near sine waves (e.g., waves having precipitous rising and/or falling portions), triangular waves, and/or complex waves. As used herein a sine wave can be generally defined as any uniform wave that is generated by a single frequency, and in particular, a wave whose amplitude is the sine of a linear function of time when plotted on a graph that plots amplitude against time. The pulses can also include one or more relatively minor perturbations or fluctuations within and/or between individual waves, such that the overall wave form is substantially as described above. Such perturbations can be desirable, for instance, in order to provide more efficacious mucus production in a manner similar to traditional hand delivered chest massages. In some embodiments, the pulse frequency module 14 may be programmed and controlled electronically to allow for the automatic timed cycling of frequencies, with the option of manual override at any frequency.

**67.** Referring to FIGS. 7-8, the fan blade 20 may include a hub 30, a base plate element 31 and a variable thickness outer wall 32. The outer wall 32 may be thinner in the region generally opposite cutout portion 22, and thicker proximate to the cutout portion. Particularly, the outer wall 32 thickness may be varied in order to statically and dynamically balance the fan blade 20. By balancing blade 20, a reduction in vibration and noise can be provided. One or more Hall effect sensors may be used with blade 20 or a valve motor to monitor and/or control rotational speed or frequency.

**68.** FIGS. 9 and 10 illustrate the pulse frequency control module 14 flowably coupled to the pressure control device 16. The pressure control device 16 may be coupled to the plate 27a in some embodiments, for example. As shown, the frequency control module 14 may interact with air flow received, for example from the air flow generator 12, before the air flow enters the pressure control device 16. FIG. 9 further illustrates the arrangement of motor 21 in communication with the frequency control module. Outlets 51, 52 may be arranged between the pressure control device 16 and the external ports 80a, 82a, for example, in order to direct air from the device 110 to a patient device, such as a patient vest.

**69.** In some embodiments, the pressure control device 16 may have a balancing chamber/manifold 50 in communication with ports 26a and 26b of module 14. The pressure control device 16 may be adapted to receive or pass air through ports 26a and 26b of pulse frequency control module 14, and effectively provide a manifold or air chamber to deliver air to the patient device and/or the atmosphere. During operation, the pressure control device 16 may receive air pulse pressure waves through ports 26a, 28a. In some embodiments, an atmospheric vent may be coupled to port 28b of the frequency control module 14. The atmospheric vent may be closed to the atmosphere when port 26a is open, and may be open to the atmosphere when port 26a is closed. The pressure control device 16 may be active or passive. For example, an active pressure control device 16 may include, for example, valves and electric solenoids in communication with an electronic controller, microprocessor, etc. A passive pressure control device 16 may include a manual pressure relief or, in a simple embodiment, the pressure control unit may include only the air chamber providing air communication between the air lines extending to the patient device and not otherwise including a pressure relief or variable pressure control.

**70.** FIG. 11 shows an air flow diagram 300 associated with the percussive pulsing therapy device of the present disclosure, such as device 110 described above. As shown, the device may have an airflow generator 12 that draws air from the atmosphere. Air from the airflow generator 12 may flow to a frequency control module 14 having a rotating blade 20 or other valve. The rotating blade 20 may be powered by motor 21. The frequency control module 14 may further have a vent 53 for releasing air back to the atmosphere. From the frequency control module 14, air may travel in the device to a pressure control device 16. The pressure control device 16 may have a balancing chamber/manifold 50. From the pressure control device 16, air may travel through outlets 51 and 52, and pass through ports 80a and 82a into airways 60 before reaching the patient device 120 through ports 80b and 82b. Generally, the patient device 120 may have a bladder, which may fill or partially fill with the air entering the patient device. Air leaving the patient device 120 may travel through ports 80b and 82b and airways 60. Air in the device 110 may be recirculated back to the pressure control device 16 at outlets 51 and 52. Alternatively, in some embodiments, a single outlet 51 may be used for air to travel from the balancing chamber/manifold 50 to port 80a, through airway 60, and into the patient device 120 through port 80b. In such embodiments, air leaving the patient device 120 may return through port 80a and recirculate back to the pressure control device 16 via airway 60 and outlet 51

**71.** FIG. 12 shows a schematic diagram 200 of a percussive pulsing therapy device of the present disclosure, such as device 110 described above. As shown, the device may have a power inlet 202 communicably coupled to a power inlet module 204. The power inlet module 204 may in turn be communicably coupled to a power supply 206 and a blower 208 generating airflow, which may be similar to the air flow generator 12 discussed with respect to FIGS. 4A-4F. As shown in FIG. 12, the blower 208 may couple via one or more airflow lines 209 to a valve assembly 218, which may have a balance block. The valve assembly 218 may be similar to the frequency control module 14 and/or pressure control device 16 described above. Moreover, the valve assembly 218 may have one or more airflow lines 220a, 220b leading to a patient device, such as a patient vest, in order to deliver pulsed and/or pressurized air to the patient. The valve assembly 218 may additionally have an exhaust line 222 in some embodiments for expelling air into the atmosphere. The power supply 206 may be communicably coupled to a central processing unit (CPU) board 210, which may be similar to the controller 140 discussed above with respect to FIG. 1. The CPU board 210 may generally have computer hardware and/or software configured to control one or more aspects of the therapy device. The CPU board may have a control panel 212 and an LCD display 214 or other display in some embodiments. The CPU board 210 may additionally couple to a stepper motor 216 or other motor, which may be similar to the motor 21 discussed above. The motor 216 may in turn communicably couple to the valve assembly 218. In other embodiments, a therapy device of the present disclosure may have additional or alternative components.

**72.** While the percussive pulsing therapy device 110 has been generally described above, a device of the present disclosure may be more specifically described in U.S. Patent No. 7,597,670, entitled "Chest Compression Apparatus, and filed August 15, 2005; U.S. Patent No. 6,958,046, entitled "Chest Compression Apparatus, and filed January 2, 2002; U.S. Patent No. 7,762,967, entitled "Chest Compression Apparatus," and filed September 12, 2006.

**73.** In use, a therapy system 100 of the present disclosure may deliver HFCC therapy to a patient, monitor therapy data, analyze the monitored data, store the monitored data, and/or provide patient guidance, encouragement, and support with respect to therapy settings, adherence, and/or other aspects. The system 100 may monitor, for example, information particular to each therapy session undergone by a patient. A therapy session may be a period of time during which the patient receives the HFCC treatment. In some cases, therapy sessions may be prescribed or regimented by a healthcare professional. For example, a patient may be directed by a doctor to undergo 30 minute HFCC therapy sessions twice daily. A patient may receive the therapy by wearing the patient device 120 and selecting one or more options via the user interface 130.

**74.** The system 100 may track or monitor particular data related to each therapy session, such as but not limited to, user identifier or patient identifier, therapy device identifier, patient device identifier, start date and time, stop date and time, and/or duration of the therapy session. Additionally, in some embodiments, the system 100 may track or monitor information related to the particular therapy administered. For example, the system 100 may track or monitor information such as, but not limited to, starting air pressure, ending air pressure, average air pressure, maximum air pressure, minimum air pressure, starting pulse frequency, ending pulse frequency, average pulse frequency, maximum pulse frequency, and/or minimum pulse frequency. The system 100 may additionally track or monitor sensed data, such as airflow data, inhalation data, exhalation data, and/or other data sensed by one or more sensors 122. The system 100 may also track or monitor any user inputs or changes to the therapy during the session. For example, the system 100 may track or monitor any pauses that the patient may take during the session and any changes in air pressure and pulse frequency during the session. The system 100 may track or monitor a total therapy time for the session, which may include a duration time minus any pause time recorded. Where the system 100 provides for preprogrammed therapy sessions, settings, or steps (i.e., intervals), the system may track or monitor any preprogrammed selections made by the patient and/or any changes to those settings. Additionally, the system 100 may record the manner in which the therapy session ended, such as by abruptly resetting or turning off the therapy device 110 or by allowing a preprogrammed time to elapse, for example.

**75.** In some embodiments, the system 100 may compile tracked or monitored data for multiple therapy sessions. The system 100 may average the data or visually represent the data in the form of graphs, charts, tables, or other representations. The system may record the number of therapy sessions tracked or monitored; the number of days over which therapy sessions have been tracked or monitored; average, maximum, or minimum duration or therapy time per session or per day; average, maximum, or minimum air pressure; average, maximum, or minimum pulse frequency; average, maximum, or minimum airflow; average, maximum, or minimum inhalation depth; and/or other metrics.

**76.** FIG. 13 shows an example of recorded therapy data for therapy sessions recorded over a period of time, according to some embodiments. As shown, data particular to each therapy session may be recorded, such as but not limited to, the date, start time, stop time, total time, therapy time, pause time, starting pressure, ending pressure, session average pressure, program setting, number of steps, and number of pauses. It may be appreciated that air pressure measurements may be actual pressure or relative pressure. In the data illustrated in FIG. 13, air pressure is shown as relative pressure readings. Additionally, data applicable to or averaged over the period of time may be calculated and/or recorded, such as but not limited to, the device identifier (serial number), days of use, average number of sessions per day, average time spent per day, average time per session, and average pressure.

**77.** As described above, therapy data for one or more therapy sessions may be stored in the database. Generally, a user may have the ability to view or access therapy session data for one or more therapy sessions. For example, a user may view current or previous therapy data via the user interface. Additionally, in some embodiments, therapy data for one or more therapy sessions may be communicated to other users and/or devices. For example, therapy data for one or more sessions may be communicated to the patient's doctor or other healthcare professional. In some embodiments, the data may be automatically emailed, uploaded or downloaded to an accessible location, or otherwise transmitted to the healthcare professional. In other embodiments, the healthcare professional may access the data remotely via, for example, a smartphone or other user interface. For example, FIG. 14 shows an example of recorded therapy data and adherence data related to a plurality of patients. A user, such as a medical professional, may have access to each patient's condition or other medical information, prescription data, therapy data including progress statistics, adherence data, and/or other data. FIGS. 15-17 illustrate detailed views of individual patient data that may be accessible to a health professional, caregiver, or other individual. The data displayed in FIGS. 13-17 may be accessible via various user interfaces. For example, in some embodiments, such patient data as that shown in FIGS. 13-17 may be available via an Internet website or portal accessible via a desktop computer, laptop computer, smartphone, tablet computer, or other web-accessible device. In other embodiments, such data may be available via any other suitable program, application, and/or device.

**78.** In some embodiments, therapy session data may be analyzed and/or compared against previous, stored, or other data. For example, therapy data from one or more therapy sessions may be compared against historical therapy data, other patients' therapy data, predetermined goals or expected therapy data, and/or other data. In at least one embodiment, measured or calculated cough rate or tidal volume data for one or more patients may be compared against recorded adherence data for one or more patients. Analysis of therapy data may additionally or alternatively include calculations, such as pressure, air volume, airflow, air speed, and/or other calculations, which may be based on one or more measured or recorded values. In at least one embodiment, data measured from an air pressure sensor on or near a patient device may be used to calculate a patient's resting inhalation pressure and/or inhalation pressure during therapy. In other embodiments, measured, recorded, monitored, or tracked therapy data may be analyzed in other ways and against other types of data.

**79.** Therapy data may generally be used to promote, encourage, guide, or otherwise help to increase patient adherence. For example, therapy data may be used to provide patient interventions related to skills training, goal setting, feedback, reinforcement, environmental prompts, and/or social support.

**80.** In some embodiments, therapy data and/or adherence data may be used to provide skills training to a patient or other system user. Skills training may include training a patient, for example, regarding effective inhalation techniques, exhalation techniques, other breathing techniques, patient device fit and adjustment, effective therapy session progression, effective pulse frequency selection or progression, effective pressure selection or progression, self-diagnosis or self-assessment of various conditions, symptoms, or other concerns, and/or other techniques or skills. Skills training interventions may include providing instructions or tutorials via the user interface, for example. Moreover, skills training interventions may include displaying or otherwise providing the patient's therapy data and/or adherence data. Skills training interventions may additionally include comparing a patient's therapy data to expected, suggested, or other therapy data to determine particular skills or aspects that may benefit from improved technique or further instruction. In some embodiments, such comparisons may be made during a therapy session such that a patient may be provided with therapy data or instructions or tips to improve skills during the session. Additionally or alternatively, therapy data or instructions, tips, or the like may be provided to the patient before or after a therapy session, or at any other suitable time, including for example, at predetermined intervals or on demand.

**81.** In some embodiments, therapy data and/or adherence data may be used to provide goal setting or action planning assistance to a patient or other system user. Users may desire to, or may generally benefit from, setting goals related to depth of inhalation, therapy duration, pulse frequency (such as a maximum, minimum, or average pulse frequency for a therapy session), air pressure (such as a maximum, minimum, or average pressure for a therapy session), frequency dwell times, cough production, sputum production, and/or other components of HFCC therapy. Goals may be set for each therapy session, or goals may be daily, weekly, monthly, or annual goals, for example. In some embodiments, the system, via the user interface for example, may prompt a user to enter one or more therapy goals. In other embodiments, the system may automatically set one or more goals for a patient based on the patient's health data, therapy data, and/or other data. In still further embodiments, one or more goals may be a community goal, for example, which may be a goal established for a plurality of users or patients. Such community goals may be set manually or automatically. A community goal may be a goal for each user or patient to meet, or may be a goal for a plurality of users to meet collectively. Other goals, which may be personal or community goals, may be set or established as well. Moreover, the system may present users with goal progress updates. Such progress updates may be provided, via the user interface for example, before, during, and/or after a therapy session. Progress updates may additionally or alternatively be provided at predetermined time intervals, such as hourly, daily, weekly, or monthly, for example. In other embodiments, progress updates may be presented at any other suitable time interval. In some embodiments, the system may provide a user with guidance to help the user meet his or her goals. Such guidance may be provided before, during, and/or after a therapy session. Additionally or alternatively, such guidance may be provided on an hourly, daily, weekly, monthly, or other suitable time interval.

**82.** In some embodiments, therapy data and/or adherence data may be used to provide feedback or reviews to a patient or other system user. Feedback may include progress updates or detailed or summarized therapy data, for example. In some embodiments, feedback may include a detailed or summarized assessment of a patient's performance during one or more therapy sessions, which may relate to the duration of the therapy session, pulse frequency (maximum, minimum, or average, for example), air pressure (maximum, minimum, or average, for example), depth of inhalation, frequency dwell times, cough production, sputum production, and/or other components of HFCC therapy. Feedback may additionally or alternatively include advice or guidance toward improving a patient's performance or therapy data or toward increasing the effectiveness of therapy sessions. Such feedback may be provided to a user, via the user interface for example, before, during and/or after a therapy session. Additionally or alternatively, such feedback may be provided on an hourly, daily, weekly, monthly, annual, or other suitable frequency or time interval.

**83.** In some embodiments, therapy data and/or adherence data may be used to provide reinforcement or consequences to a patient or other system user. Reinforcement may include altering therapy session parameters or settings based on a patient's current or historical therapy data or performance. For example, pulse frequency, air pressure, therapy duration, or other parameters may be altered. In at least one embodiment, for example, where a patient's therapy data during a therapy session indicate that the session is particularly effective in treating the patient's condition, the duration of that particular therapy session may be decreased, and/or a recommendation may be made to decrease the duration of future therapy sessions. Similarly, where therapy data indicates that a current or past therapy session is particularly ineffective, the duration, air pressure, and/or pulse frequency may be increased and/or a recommendation may be made to increase such parameters for future therapy sessions. In some embodiments, therapy settings or parameters may be altered automatically in response to the patient's therapy data. In other embodiments, the system may provide suggestions for altering parameters. Parameter changes or suggestions for parameter changes may be made based on a particular therapy data metric is achieved, or where one or more therapy metrics reaches, exceeds, or falls below one or more predetermined thresholds. For example, where a sensor indicates that airflow is above a particular threshold during a therapy session, the system may reduce the duration of the therapy session or suggest reducing the duration of the therapy session. Moreover, reinforcement may include presenting a user with clinical outcomes. For example, the system may present the user with one or more likely potential clinical outcomes based on the patient's therapy data. A clinical outcome may show, for example, the likely therapy effectiveness, airflow improvement, sputum production, or symptom reduction that may result if the patient continues with current, recent, or average therapy parameters, or increases or decreases such parameters.

**84.** In some embodiments, reinforcement may include determining and/or reporting incidents of "spoofing." Spoofing may occur where a patient attempts to trick the system into recording a therapy session, when in fact, the patient did not receive the therapy session. For example, the user may allow the system to operate a therapy session by sending pressurized, pulsed air to a patient device, while the user is not wearing the device. Or the user may remove the device partially through a therapy session, and allow the therapy session to continue running. Thus, the system may record or log that a therapy session was completed, but the user may not have actually received the therapy. The system may operate to detect spoofing efforts by recording and/or analyzing airflow data. For example, a sensor configured to detect inhaled and/or exhaled air from the patient's mouth or nose may indicate little to no inhaled or exhaled air for a period of time, which may suggest spoofing. Similarly, a sensor configured to detect patient device pressure may show different peak pressures when a patient is wearing the patient device and when a patient is not wearing the device. A peak pressure indicating that the device is not being worn may suggest spoofing. Data suggesting spoofing may be communicated to one or more system users. For example, the system may alert the patient or another user via the user interface or another device that therapy is not being received. In some embodiments, the system may alert a healthcare professional that the patient may not be receiving the appropriate therapy.

**85.** In some embodiments, therapy data and/or adherence data may be used to provide environmental prompts to a patient or other system user. Environmental prompts may include therapy data, advice or tips, reminders, encouragements, or other data displayed to a user via, for example, the user interface. In some embodiments, environmental prompts may be displayed in real time during a therapy session. Environmental prompts may be configured to encourage a patient to, for example, complete a therapy session, increase the effectiveness or intensity of a therapy session, and/or achieve a goal. One example of an environmental prompt may be a graph or chart showing target inhalation goals and progress in real time displayed during a therapy session. Another example of an environmental prompt may be a countdown clock counting down to the end of a therapy session displayed in real time during the therapy session. As another example of an environmental prompt, when a user reaches a target pulse frequency during a therapy session, a reminder may be displayed to inform the user how long, based on particular therapy data, the user should dwell on the target frequency in order to increase effectiveness. As yet another example of an environmental prompt, where the system detects that a patient is not breathing deeply enough or coughing frequently enough for high efficiency of the therapy, a message may be displayed or a sound may be played, for example, to remind the user to breathe deeply or to cough.

**86.** In some embodiments, therapy data and/or adherence data may be used to provide social support or comparison to a patient or other system user. Social support may include comparisons between a patient's therapy data and therapy data for one or more other patients. For example, the system may display, via the user interface for example, a chart or graph showing how a patient's therapy progress compares to that of other patients with similar health data or conditions. In some embodiments, social support may include providing an option for a patient to share his or her therapy data with other users, friends, family members, or others. For example, after a therapy session, the system may display a patient's therapy data, progress or improvements, and/or goal meeting status, and provide the option to share the information. The patient may have the option to share the data with particular individuals selected or entered by the patient, such as the patient's friends, family, or healthcare professionals. In some embodiments, the patient may have the option to share the data with a community message board, such as a message board for other system users with similar conditions; as an email, text, or other message; on an existing social media platform such as Facebook or Twitter; and/or via another communication platform. In some embodiments, users may have the option to comment on other patients' shared data. For example, if a first patient shares data related to achieving a therapy goal, a second user may have the option to view the shared data and send or post an encouraging message to the first user.

**87.** In some embodiments, therapy data and/or adherence data may be used to provide alternative or additional interventions to encourage and/or assist patients in adhering to a prescribed or suggested therapy regimen or schedule.

**88.** As described above, a user, such as a patient or caregiver, may have access to therapy and/or adherence data via a user interface. For example, in some embodiments, a mobile application may provide a user, such as a patient or caregiver, with updated therapy data and/or adherence data. FIGS. 18A-18E illustrate examples of a mobile user interface providing access to detailed therapy data and adherence data for a patient. As shown, the interface may display therapy and/or adherence data, including any therapy data and/or adherence data discussed above, for individual therapy sessions, and/or data trends over a plurality of sessions. The interface may additionally allow patients to view and/or schedule therapy sessions. Moreover, in some embodiments, a user may set and/or receive reminders related to scheduled therapy sessions, and/or other reminders as described above with respect to adherence, such as cough reminders, reminders to adjust one or more therapy settings, goal reminders, and/or other reminders via the user interface. In some embodiments, the mobile application may provide options for adjusting one or more settings on the therapy device in real time. For example, a user may adjust a pulse frequency, pressure setting, and/or therapy timer of the therapy device via the mobile application. Additionally or alternatively, a user may have access to the data via other interfaces, including via other devices and/or programs or applications. For example, as described above, a user may access adherence and/or therapy data via a user interface arranged on, in, or otherwise in communication with the therapy device itself. For example, the user interface may include a monitor, such as a touch screen monitor arranged on the therapy device. The navigable screens of such a user interface may be similar to those shown in FIGS. 18A-18E with respect to a mobile application interface.

**89.** In some embodiments, a mobile application and/or other user interface may be used as a diagnostic and/or progress tool. For example, a mobile application may use a microphone and/or other sensor on a smartphone or smart watch to evaluate the sound of a patient's cough and/or breathing. In some embodiments, the interface may provide diagnostic information based on the sensed information and/or one or more recommendations. For example, the interface may provide a therapy setting recommendation, such as a recommendation for a user to increase a pulse frequency and/or pulse pressure on the therapy device where it is determined based on the user's cough that the current therapy session is not desirably effective. Further, in some embodiments, the user interface may automatically cause the change in therapy setting to occur in real time.

**90.** For purposes of this disclosure, any system described herein may include any instrumentality or aggregate of instrumentalities operable to compute, calculate, determine, classify, process, transmit, receive, retrieve, originate, switch, store, display, communicate, manifest, detect, record, reproduce, handle, or utilize any form of information, intelligence, or data for business, scientific, control, or other purposes. For example, a system or any portion thereof may be a minicomputer, mainframe computer, personal computer (e.g., desktop or laptop), tablet computer, mobile device (e.g., personal digital assistant (PDA) or smart phone) or other hand-held computing device, server (e.g., blade server or rack server), a network storage device, or any other suitable device or combination of devices and may vary in size, shape, performance, functionality, and price. A system may include volatile memory (e.g., random access memory (RAM)), one or more processing resources such as a central processing unit (CPU) or hardware or software control logic, ROM, and/or other types of nonvolatile memory (e.g., EPROM, EEPROM, etc.). A basic input/output system (BIOS) can be stored in the non-volatile memory (e.g., ROM), and may include basic routines facilitating communication of data and signals between components within the system. The volatile memory may additionally include a high-speed RAM, such as static RAM for caching data.

**91.** Additional components of a system may include one or more disk drives or one or more mass storage devices, one or more network ports for communicating with external devices as well as various input and output (I/O) devices, such as a keyboard, a mouse, touchscreen and/or a video display. Mass storage devices may include, but are not limited to, a hard disk drive, floppy disk drive, CD-ROM drive, smart drive, flash drive, or other types of non-volatile data storage, a plurality of storage devices, a storage subsystem, or any combination of storage devices. A storage interface may be provided for interfacing with mass storage devices, for example, a storage subsystem. The storage interface may include any suitable interface technology, such as EIDE, ATA, SATA, and IEEE 1394. A system may include what is referred to as a user interface for interacting with the system, which may generally include a display, mouse or other cursor control device, keyboard, button, touchpad, touch screen, stylus, remote control (such as an infrared remote control), microphone, camera, video recorder, gesture systems (e.g., eye movement, head movement, etc.), speaker, LED, light, joystick, game pad, switch, buzzer, bell, and/or other user input/output device for communicating with one or more users or for entering information into the system. These and other devices for interacting with the system may be connected to the system through I/O device interface(s) via a system bus, but can be connected by other interfaces such as a parallel port, IEEE 1394 serial port, a game port, a USB port, an IR interface, etc. Output devices may include any type of device for presenting information to a user, including but not limited to, a computer monitor, flat-screen display, or other visual display, a printer, and/or speakers or any other device for providing information in audio form, such as a telephone, a plurality of output devices, or any combination of output devices.

**92.** A system may also include one or more buses operable to transmit communications between the various hardware components. A system bus may be any of several types of bus structure that can further interconnect, for example, to a memory bus (with or without a memory controller) and/or a peripheral bus (e.g., PCI, PCIe, AGP, LPC, etc.) using any of a variety of commercially available bus architectures.

**93.** One or more programs or applications, such as a web browser and/or other executable applications, may be stored in one or more of the system data storage devices. Generally, programs may include routines, methods, data structures, other software components, etc., that perform particular tasks or implement particular abstract data types. Programs or applications may be loaded in part or in whole into a main memory or processor during execution by the processor. One or more processors may execute applications or programs to run systems or methods of the present disclosure, or portions thereof, stored as executable programs or program code in the memory, or received from the Internet or other network. Any commercial or freeware web browser or other application capable of retrieving content from a network and displaying pages or screens may be used. In some embodiments, a customized application may be used to access, display, and update information. A user may interact with the system, programs, and data stored thereon or accessible thereto using any one or more of the input and output devices described above.

**94.** A system of the present disclosure can operate in a networked environment using logical connections via a wired and/or wireless communications subsystem to one or more networks and/or other computers. Other computers can include, but are not limited to, workstations, servers, routers, personal computers, microprocessor-based entertainment appliances, peer devices, or other common network nodes, and may generally include many or all of the elements described above. Logical connections may include wired and/or wireless connectivity to a local area network (LAN), a wide area network (WAN), hotspot, a global communications network, such as the Internet, and so on. The system may be operable to communicate with wired and/or wireless devices or other processing entities using, for example, radio technologies, such as the IEEE 802.xx family of standards, and includes at least Wi-Fi (wireless fidelity), WiMax, and Bluetooth wireless technologies. Communications can be made via a predefined structure as with a conventional network or via an ad hoc communication between at least two devices.

**95.** Hardware and software components of the present disclosure, as discussed herein, may be integral portions of a single computer or server or may be connected parts of a computer network. The hardware and software components may be located within a single location or, in other embodiments, portions of the hardware and software components may be divided among a plurality of locations and connected directly or through a global computer information network, such as the Internet. Accordingly, aspects of the various embodiments of the present disclosure can be practiced in distributed computing environments where certain tasks are performed by remote processing devices that are linked through a communications network. In such a distributed computing environment, program modules may be located in local and/or remote storage and/or memory systems.

**96.** As will be appreciated by one of skill in the art, the various embodiments of the present disclosure may be embodied as a method (including, for example, a computer-implemented process, a business process, and/or any other process), apparatus (including, for example, a system, machine, device, computer program product, and/or the like), or a combination of the foregoing. Accordingly, embodiments of the present disclosure may take the form of an entirely hardware embodiment, an entirely software embodiment (including firmware, middleware, microcode, hardware description languages, etc.), or an embodiment combining software and hardware aspects. Furthermore, embodiments of the present disclosure may take the form of a computer program product on a computer-readable medium or computer-readable storage medium, having computer-executable program code embodied in the medium, that define processes or methods described herein. A processor or processors may perform the necessary tasks defined by the computer-executable program code. Computer-executable program code for carrying out operations of embodiments of the present disclosure may be written in an object oriented, scripted or unscripted programming language such as Java, Perl, PHP, Visual Basic, Smalltalk, C++, or the like. However, the computer program code for carrying out operations of embodiments of the present disclosure may also be written in conventional procedural programming languages, such as the C programming language or similar programming languages. A code segment may represent a procedure, a function, a subprogram, a program, a routine, a subroutine, a module, an object, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

**97.** In the context of this document, a computer readable medium may be any medium that can contain, store, communicate, or transport the program for use by or in connection with the systems disclosed herein. The computer-executable program code may be transmitted using any appropriate medium, including but not limited to the Internet, optical fiber cable, radio frequency (RF) signals or other wireless signals, or other mediums. The computer readable medium may be, for example but is not limited to, an electronic, magnetic, optical, electromagnetic, infrared, or semiconductor system, apparatus, or device. More specific examples of suitable computer readable medium include, but are not limited to, an electrical connection having one or more wires or a tangible storage medium such as a portable computer diskette, a hard disk, a random access memory (RAM), a read-only memory (ROM), an erasable programmable read-only memory (EPROM or Flash memory), a compact disc read-only memory (CD-ROM), or other optical or magnetic storage device. Computer-readable media includes, but is not to be confused with, computer-readable storage medium, which is intended to cover all physical, non-transitory, or similar embodiments of computer-readable media.

**98.** Various embodiments of the present disclosure may be described herein with reference to flowchart illustrations and/or block diagrams of methods, apparatus (systems), and computer program products. It is understood that each block of the flowchart illustrations and/or block diagrams, and/or combinations of blocks in the flowchart illustrations and/or block diagrams, can be implemented by computer-executable program code portions. These computer-executable program code portions may be provided to a processor of a general purpose computer, special purpose computer, or other programmable data processing apparatus to produce a particular machine, such that the code portions, which execute via the processor of the computer or other programmable data processing apparatus, create mechanisms for implementing the functions/acts specified in the flowchart and/or block diagram block or blocks. Alternatively, computer program implemented steps or acts may be combined with operator or human implemented steps or acts in order to carry out an embodiment of the invention.

**99.** Additionally, although a flowchart or block diagram may illustrate a method as comprising sequential steps or a process as having a particular order of operations, many of the steps or operations in the flowchart(s) or block diagram(s) illustrated herein can be performed in parallel or concurrently, and the flowchart(s) or block diagram(s) should be read in the context of the various embodiments of the present disclosure. In addition, the order of the method steps or process operations illustrated in a flowchart or block diagram may be rearranged for some embodiments. Similarly, a method or process illustrated in a flow chart or block diagram could have additional steps or operations not included therein or fewer steps or operations than those shown. Moreover, a method step may correspond to a method, a function, a procedure, a subroutine, a subprogram, etc.

**100.** As used herein, the terms "substantially" or "generally" refer to the complete or nearly complete extent or degree of an action, characteristic, property, state, structure, item, or result. For example, an object that is "substantially" or "generally" enclosed would mean that the object is either completely enclosed or nearly completely enclosed. The exact allowable degree of deviation from absolute completeness may in some cases depend on the specific context. However, generally speaking, the nearness of completion will be so as to have generally the same overall result as if absolute and total completion were obtained. The use of "substantially" or "generally" is equally applicable when used in a negative connotation to refer to the complete or near complete lack of an action, characteristic, property, state, structure, item, or result. For example, an element, combination, embodiment, or composition that is "substantially free of' or "generally free of' an element may still actually contain such element as long as there is generally no significant effect thereof.

**101.**

## Claims

1. A system (100) for providing high frequency chest compression therapy, comprising:
a patient device (120) comprising a vest configured to be worn by a patient, the vest housing an air bladder;
a therapy device (110) flowably coupled to the patient device and providing percussive pulsed air to the patient device, the percussive pulsed air having a pressure and a frequency;
a database (150) storing therapy data recorded for one or more therapy sessions;
a user interface (130) for accessing therapy data in the database; and
a controller (140) comprising:
a device control module (142) for controlling parameters of the therapy device, the parameters comprising the pressure and frequency of the percussive pulsed air;
a therapy monitoring module (144) for monitoring and recording therapy data during a therapy session, the therapy data comprising air pressure data,
**characterized by**:
a data analysis module (146) for analyzing the therapy data and configured to determine whether different peak pressures exist when the patient is wearing the patient device and when the patient is not wearing the patient device based on the air pressure data by comparing the recorded therapy data to at least one of expected therapy data, regimented therapy data, historical therapy data, and therapy data for one or more other patients; and
an alerts module (148) configured for alerting the patient via the user interface that the therapy is not being received, and/or configured for alerting a healthcare professional that the patient may not be receiving an appropriate therapy, based on a determination result of the data analysis module.

2. The system of claim 1, wherein the alerts module is further configured for initiating user alerts for providing a social support intervention configured to provide a sharing option whereby the patient can share recorded therapy data.

3. The system of any preceding claim, further comprising at least one of a patient device pressure sensor, a spirometer, and a pneumotachometer.

4. The system of any preceding claim, wherein the user interface comprises a touch screen display.

5. The system of any preceding claim, wherein the recorded therapy data is communicated to a healthcare professional.

6. A method for increasing patient adherence to a high frequency chest compression therapy regimen, the method comprising:
accessing in a database 150, recorded therapy data for one or more therapy sessions of a patient, the therapy data comprising air pressure data,
**characterized by**:
analyzing the therapy data and determining whether different peak pressures exist when the patient was wearing the patient device and when the patient was not wearing the patient device based on the air pressure data by comparing the recorded therapy data to at least one of expected therapy data, regimented therapy data, historical therapy data, and therapy data for one or more other patients; and
alerting the patient via the user interface that the therapy has not been received, and/or alerting a healthcare professional that the patient may not have received an appropriate therapy, based on a determination result.

7. The method of claim 6, further comprising communicating the recorded therapy data to a healthcare professional.

## Patentansprüche

1. Ein System (100) zum Bereitstellen einer Hochfrequenz-Brustkompressionstherapie,das Folgendes umfassend:
ein Patientengerät (120) mit einer vom Patienten zu tragenden Weste,
wobei die Weste eine Luftblase enthält;
ein Therapiegerät (110), das fließend mit dem Patientengerät verbunden ist und dem Patientengerät stoßgepulste Luft zuführt,
wobei die stoßgepulste Luft einen Druck und eine Frequenz aufweist;
eine Datenbank (150), in der die für mindestens eine Therapiesitzung aufgezeichneten Therapiedaten gespeichert werden;
eine Benutzerschnittstelle (130) für das Zugreifen auf die Therapiedaten in der Datenbank; und
eine Steuerung (140), die Folgendes umfasst:
ein Gerätesteuerungsmodul (142) zum Steuern der Parameter des Therapiegeräts,
wobei die Parameter den Druck und die Frequenz der stoßgepulsten Luft umfassen;
ein Therapieüberwachungsmodul (144) zum Überwachen und Aufzeichnen der Therapiedaten während einer Therapiesitzung,
wobei die Therapiedaten Luftdruckdaten umfassen,
**gekennzeichnet durch**:
ein Datenanalysemodul (146) zum Analysieren der Therapiedaten und um beruhend auf den Luftdruckdaten zu ermitteln, ob unterschiedliche Spitzendruckwerte vorliegen, wenn der Patient das Gerät trägt,
und wenn der Patient das Gerät nicht trägt, indem die aufgezeichneten Therapiedaten mit mindestens einem der folgenden Elemente verglichen wird: den erwarteten Therapiedaten, den reglementierten Therapiedaten, den historischen Therapiedaten und den Therapiedaten mindestens eines anderen Patienten;
und
ein Warnmodul (148), das beruhend auf dem ermittelten Ergebnis des Datenanalysemoduls folgende Schritte durchführt: Warnen des Patienten über die Benutzerschnittstelle, wenn die Therapie nicht abgerufen wurde, und/oder
Warnen einer medizinischen Fachkraft, wenn der Patient möglicherweise keine geeignete Therapie erhält.

2. Das System gemäß Anspruch 1, wobei das Warnmodul zudem Benutzerwarnungen auslöst, um eine soziale Unterstützungsintervention bereitzustellen, die über eine Freigabeoption bereitstellt, sodass der Patient die aufgezeichneten Therapiedaten freigeben kann.

3. Das System gemäß einem der vorherigen Ansprüche, das zudem mindestens einen Drucksensor für das Patientengerät, ein Spirometer und ein Pneumotachometer umfasst.

4. Das System gemäß einem der vorherigen Ansprüche, wobei die Benutzerschnittstelle eine Touchscreen-Anzeige umfasst.

5. Das System gemäß einem der vorherigen Ansprüche, wobei die aufgezeichneten Therapiedaten an eine medizinische Fachkraft übertragen werden.

6. Eine Methode zum Optimieren der Einhaltung einer Hochfrequenz-Brustkompressionstherapie durch den Patienten,
wobei die Methode folgende Schritte umfasst: Zugreifen auf in einer Datenbank 150 aufgezeichnete Therapiedaten für mindestens eine Therapiesitzung; wobei die Therapiedaten Luftdruckdaten umfassen,
**gekennzeichnet durch**:
Analysieren der Therapiedaten und beruhend auf den Luftdruckdaten Ermitteln, ob unterschiedliche Spitzendruckwerte vorliegen, wenn der Patient das Gerät getragen hat, und wenn der Patient das Gerät nicht getragen hat, indem die aufgezeichneten Therapiedaten mit mindestens einem der folgenden Elemente verglichen wird: den erwarteten Therapiedaten, den reglementierten Therapiedaten, den historischen Therapiedaten und den Therapiedaten mindestens eines anderen Patienten; und
beruhend auf dem ermittelten Ergebnis Warnen des Patienten über die Benutzerschnittstelle, wenn die Therapie nicht erhalten wurde, und/oder Warnen einer medizinischen Fachkraft, wenn der Patient möglicherweise keine geeignete Therapie erhalten hat.

7. Die Methode gemäß Anspruch 6, die zudem das Übertragen der aufgezeichneten Therapiedaten an eine medizinische Fachkraft umfasst.

## Revendications

1. Système (100) de fourniture d'une thérapie de compression thoracique à haute fréquence, comprenant :
un dispositif patient (120) comprenant un gilet conçu pour être porté par un patient, le gilet logeant une vessie pneumatique ;
un dispositif thérapeutique (110) couplé de manière à pouvoir s'écouler au dispositif patient et fournissant de l'air pulsé à percussion au dispositif patient, l'air pulsé à percussion présentant une pression et une fréquence ;
une base de données (150) mémorisant des données thérapeutiques enregistrées pour au moins une session thérapeutique ;
une interface utilisateur (130) permettant d'accéder aux données thérapeutiques dans la base de données ;
et un dispositif de commande (140) comprenant :
un module de commande de dispositif (142) permettant de commander des paramètres du dispositif thérapeutique, les paramètres comprenant la pression et la fréquence de l'air pulsé à percussion ;
un module de surveillance thérapeutique (144) permettant de surveiller et d'enregistrer des données thérapeutiques pendant une session thérapeutique, les données thérapeutiques comprenant des données de pression d'air,
**caractérisé par** :
un module d'analyse de données (146) permettant d'analyser les données thérapeutiques et conçu pour déterminer s'il existe différentes pressions de crête lorsque le patient porte le dispositif patient et
lorsque le patient ne porte pas le dispositif patient en fonction des données de pression d'air par comparaison des données thérapeutiques enregistrées avec des données thérapeutiques attendues et/ou des données thérapeutiques réglementées et/ou des données thérapeutiques historiques et/ou des données thérapeutiques pour au moins un autre patient ; et
un module d'alertes (148) conçu pour alerter le patient par l'intermédiaire de l'interface utilisateur que la thérapie n'est pas reçue, et/ou conçu pour alerter un professionnel de la santé que le patient peut ne pas recevoir une thérapie appropriée, en fonction d'un résultat de détermination du module d'analyse de données.

2. Système selon la revendication 1, dans lequel le module d'alertes est en outre conçu pour lancer des alertes utilisateur pour fournir une intervention de soutien social conçue pour fournir une option de partage, le patient pouvant partager des données thérapeutiques enregistrées.

3. Système selon l'une quelconque des revendications précédentes, comprenant en outre un capteur de pression de dispositif patient et/ou un spiromètre et/ou un pneumotachomètre.

4. Système selon l'une quelconque des revendications précédentes, dans lequel l'interface utilisateur comprend un écran tactile.

5. Système selon l'une quelconque des revendications précédentes, dans lequel les données thérapeutiques enregistrées sont communiquées à un professionnel de la santé.

6. Procédé d'augmentation de l'adhésion d'un patient à un régime thérapeutique de compression thoracique à haute fréquence, le procédé comprenant :
l'accès dans une base de données (150) aux données thérapeutiques enregistrées pour au moins une séance thérapeutique d'un patient, les données thérapeutiques comprenant des données de pression d'air, **caractérisée par** :
l'analyse des données thérapeutiques et la détermination s'il existe différentes pressions de crête lorsque le patient portait le dispositif patient et lorsque le patient ne portait pas le dispositif patient en fonction des données de pression d'air par comparaison des données thérapeutiques enregistrées aux données thérapeutiques attendues et/ou aux données thérapeutiques réglementées et/ou aux données thérapeutiques historiques et/ou aux données thérapeutiques pour au moins un autre patient ; et
l'alerte du patient par l'intermédiaire de l'interface utilisateur que la thérapie n'a pas été reçue, et/ou l'alerte d'un professionnel de la santé que le patient peut ne pas avoir reçu une thérapie appropriée, en fonction d'un résultat de la détermination.

7. Procédé selon la revendication 6, comprenant en outre la communication des données thérapeutiques enregistrées à un professionnel de la santé
